# EUROPEAN PATENT APPLICATION

(11) **EP 4 248 982 A1**
(43) Date of publication of application: **27.09.2023**
(21) Application number: 21893917.1
(22) Date of filing: 17.11.2021
(51) Int. Cl.: A61K 36/82, A61K 33/14, A61P 39/06, A61P 39/00

(54) **ANTI-AGING REGENERATION COMPOSITION**

(30) Priority: 19.11.2020 CN 202011299050
(71) Applicant: Xu, Haohan, Shenzhen, Guangdong 518000 (CN)
(72) Inventor: Xu, Haohan, Shenzhen, Guangdong 518000 (CN)
(74) Representative: ZHAOffice SPRL
(86) International application number: PCT/CN2021/131068
(87) International publication number: WO 2022/105770

(57) **Abstract**

This application relates to an anti-aging regeneration formula, a preparation method and its application, which can repair the damage of the human nervous system, induce the regenerative ability of the human body, and has the potential of inhibiting pain, inhibiting inflammation and prolonging life, belonging to the field of biomedicine.

## Description

### Technical Field

This application pertains to the medical field ;it relates to a formula and a preparation method thereof, encompasses a formula capable of activating the regenerative repair function within the human body. Inflammation and aging detrimentally impact the elderly, slowing down their rate of recovery and rendering the restoration of their youthful physical state seemingly unattainable. Researchers have unveiled a method to initiate the body's regenerative repair function, which exhibits a close association with the human nervous system. The formula elucidated in this application demonstrates the potential to expedite the recovery rate of elderly individuals

. This application relates to a formula in prevention or treatment, combining anti-aging and regenerative repair. The aging process leads to the decline of bodily tissue and organ functions, often accompanied by inflammation and tumors. Therefore, the suppression of inflammation and tumors is vital for anti-aging purposes. Furthermore, enhancing the concentration of chloride ions, sodium ions, and various small molecules of neurotransmitters in the nervous system plays a pivotal role in repair and regeneration. This application is firmly positioned within the medical field.

### . Background Technology

. Regenerative medicine is a widely recognized concept that endeavors to enhance the body's self-repair mechanisms to a clinically meaningful extent. Extensive research suggests that tackling the challenges of aging predominantly revolves around three key aspects: regenerative repair, degenerative diseases affecting the central nervous system, and the management of inflammation and cancer_{∘}

. Currently, there are three internationally recognized regenerative medical technologies. The first one is cell therapy, which involves identifying appropriate cells and implanting them into the human body. For instance, stem cells obtained from hip joints can be directly injected into the heart. Clinical studies have demonstrated that cell therapy can help restore some patients with mild symptoms to a symptom-free state. Moreover, the liquid obtained from liposuction procedures contains a substantial number of stem cells, and these isolated stem cells have the ability to regenerate neurons.

The second type of regenerative medical technology utilizes foreign materials to stimulate the body's repair mechanisms. For example, scientists have utilized pig intestines due to their excellent healing capabilities. When placed in different parts of the human body, pig intestines can trigger appropriate repair responses in various tissues, resulting in positive treatment outcomes such as long-term ulcer healing, tissue reconnection in cases of fractures, and successful regeneration of missing fingertips in elderly individuals. Japan has been at the forefront of stem cell treatments and research. Initially, hyaluronic acid preparations were injected into joints, but they proved ineffective. Subsequently, the injection of adipose-derived stem cells cultured outside the body showed promising results.

The third type of regenerative medical technology involves the utilization of devices that alleviate the body's burden and facilitate self-healing processes.

In the field of cancer treatment, several current technologies are available, including chemotherapy, radiation therapy, and immunotherapy. Despite these significant advancements, the mortality rate among cancer patients remains alarmingly high, especially for malignant tumors with low 5-year survival rates. A prevailing belief among medical researchers is that cancer is fundamentally an immune system disorder, characterized by the failure of the immune system to identify and control the spread and migration of malignant tumors.

During the course of the experiment, careful observations and analyses have revealed a potential mechanism underlying the dissemination and migration of malignant tumors. In this particular scenario, it has been observed that there is a simultaneous elevation in the concentration of sodium ions and amino acids. Furthermore, this process is accompanied by the manipulation of signaling pathways within the human nervous system, thereby facilitating the intricate process of tumor spread and migration.

Based on the available literature, multiple studies have provided evidence for the correlation between alterations in cell membrane potential and cell mitosis activity. Elevated intracellular levels of cations have been associated with a greater likelihood of malignancy. It has been observed that the percentage of Na+ concentration gradually increases from normal tissues to benign lesions or transitional tumors, and finally to malignant tumors. Specifically, the percentage of sodium ion concentration shows a gradient trend of 0.05% in normal tissues, 0.05% to 0.07% in benign lesions or transitional tumors, and 0.15% to 0.2% in malignant tumors. Sodium ion concentration in tumors and the surrounding tissues is approximately 50% to 60% higher than that in normal tissues. Malignant tumor cells exhibit an extracellular sodium ion concentration of 451 mmol/l, which closely resembles the extracellular sodium ion concentration found in regenerative neuronal cells of animals like octopuses and squids, which is around 440 mmol/l. Furthermore, malignant tumor cells demonstrate amino acid absorption rates that are more than ten times higher than those of normal cells.

Researchers have proposed a hypothesis suggesting that the simultaneous elevation of sodium ion concentration outside nervous cell membrane and the concentration of various amino acids in the human nervous system, reaching or surpassing levels found in the sodium ion concentration outside nervous cell membrane of octopuses, could potentially activate the regenerative capacity of the human body. The accumulation of this regenerative ability holds the potential for tissue and organ regeneration and repair. It is believed that this regenerative capability is achieved through the regulated control of neurotransmitter concentrations within the human nervous system. According to this hypothesis, cancer cells, particularly malignant tumors, increase the concentrations of sodium ions and amino acids, resulting in alterations in neurotransmitter concentrations within the nervous system. As a result, cancer cells can manipulate the human nervous system and control the body's electrical signaling system, thus facilitating their own dissemination and migration. This suggests that the migration and progression of cancer cells cannot be completely suppressed by immunotherapy alone, emphasizing the need to address cancer through the combined actions of the nervous and immune systems. Similarly, based on this hypothesis, it is suggested that viruses, bacteria, and other microorganisms induce inflammation, leading to an increase in sodium ion concentration ranging from 200mmol/l to 400mmol/l within inflamed areas. These pathogens stimulate the production of mucoproteins and inflammatory byproducts, which include amino acid components. Consequently, changes in neurotransmitter concentrations occur within the nervous system at the sites of inflammation. Thus, inflammation exerts control over the human nervous system and influences the body's signaling system, contributing to the spread of pain and inflammation.

. Human beings possess inherent regenerative capabilities that have remained dormant. Experimental findings have unveiled a crucial correlation between the regenerative capacity of the human body and the nervous system. The human nervous system actively absorbs specially formulated solutions, converting the assimilated small molecular components into a variety of neurotransmitters. This remarkable process not only facilitates rapid and precise repair of damaged nervous tissue, elimination of inflammatory mucus, and alleviation of pain but also enables the regeneration and restoration of human tissue and organs under the influence of the nervous system.

The regeneration process involves the swift breakdown and elimination of inflammatory pathogens, uric acid, free radicals, and other substances. Subsequently, guided by electrical signals from the nervous system, fat cells, stem cells, and regenerative nutrients adeptly and naturally converge near the site of injury, fostering the regeneration of human tissue. Throughout this process, it is imperative to consistently and regularly administer the formulated solution on a daily basis. The regenerative effects are characterized by scar-free healing, absence of pain and inflammation, and a regenerated outcome that strikingly resembles the original healthy tissue.

. Distinctions between this application and the forefront of international regenerative medicine: This application sets itself apart in several ways. Firstly, it eliminates the need for ex vivo cultivation or injection of stem cells, making the process more convenient and accessible. Secondly, it avoids the requirement for small intestinal mucosa, reducing the level of artificial intervention. Instead, it focuses on targeted application to specific areas such as the skin, mucous membranes, pulmonary mucosa, intestinal mucosa, upper and lower eyelids, and retina. The formulated solution can be administered through nebulization, topical application, or immersion.

The key mechanism of this approach lies in the active absorption through the nervous system. By absorbing the formulated solution, it effectively eliminates inflammatory mucus and disrupts or replaces pain signals, resulting in pain inhibition and facilitating tissue regeneration and repair. The benefits of this regenerative method include its non-invasiveness, cost-effectiveness, reduction or elimination of pain, rapid expulsion of inflammatory mucus, and minimal side effects. In essence, this invention accelerates the body's natural self-repair process, ensuring efficient and cost-effective replication and regeneration of tissues.

In contrast to central nervous system drugs, such as AD drugs, which have exhibited a high clinical failure rate of 99% in the past 20 years, this application shows promise in inhibiting brain disorders, eye diseases, central nervous system diseases, and pathological neuropathic pain diseases. These conditions have been challenging to address due to their complex pathogenesis and limited understanding, resulting in a lack of new drugs in the market for the past two decades.

. The combination of anti-aging and regeneration is of significant importance.

Industrial practicality: The preparation method utilizes a physical approach wherein tea are boiled in water, and high-concentration solutions of these components are extracted. These solutions are then combined with sodium chloride to create the formulated solution, known as X solution. The concentration of sodium chloride in the X solution reaches approximately 26.47% in water, representing its saturation point. At this saturation point, the solubility is approximately 36 (with minor variations based on temperature), ensuring the industrial practicality and stability of the formulated solution using the saturated concentration of sodium chloride. On one hand, amino acids have a relatively short half-life. However, saturating them with sodium chloride significantly prolongs their half-life, thereby extending the therapeutic efficacy and disease-inhibiting capabilities of small molecular components, including amino acids, in the X solution. On the other hand, saturating the sodium chloride solution inhibits the absorption of other airborne components, preventing the formation of nitric acid and ammonia compounds within the X solution. This contributes to the stability of its composition.

The extraction method for components tea involves a physical process. After boiling in water, a substantial amount of tea is added to yield a high-concentration solution of these components. This solution contains over 500 effective components found in tea, with many of them being small molecular components at the nanoscale.

### .Contents of the Invention

. This anti-aging and regenerative formula boasts minimal side effects, excellent efficacy, and a straightforward application process.

.an anti-aging and regenerative formula the formula includes tea components and sodium chloride, potassium chloride, and any combination of sodium salts, potassium salts, and calcium salts permitted by any pharmaceutical. Tea has more than 500 ingredients, 500 tea ingredients, Vitamins (C, B1, B2, B3, B5, B6, B11, B12, P1, H, A, D, E, K Etc.), amino acids (theanine, aspartate, serine, glutamic acid, glycine, tryptophan, valine, histidine, arginine, threonine, alanine, proline, cysteine, tyrosine, methionine, lysine, isoleucine, leucine, phenylalanine, etc.), alkaloids (caffeine, tealeine, theobromine, etc.), tea polyphenols (catechin, flavonoids, flowers, etc.) Pigments, phenolic acids, etc.), inorganic substances (potassium, phosphorus, calcium, magnesium, iron, sulfur, aluminum, fluorine, etc.), organic acids, lipids (phospholipids, triglycerides, sulfur lipids, glycolipids), pigments (chlorophyll, anthocyanin, carotene, lutein, etc.), aromatic substances, enzymes. One of the formulation of the solution includes Tieguanyin tea, sodium chloride, and water. the tea is boiled to produce a high concentration of tea, while simultaneously achieving a state of saturation in the presence of sodium chloride (reaching the maximum saturation level of sodium chloride dissolution).

Wherein said formulation solution possesses strong lipophilic properties , enabling efficient decomposition of polycyclic aromatic hydrocarbons such as benzo[a]pyrene and disruption of the viscosity of heavy oil fouling. Wherein said formulation solution is applied topically to various human organs, including the skin, eyes, mucous membranes, oral cavity, intestines, and hair. The human skin, mucous membranes, eyes, and lung membranes exhibit rapid absorption of the formulation solution, especially when actively absorbed by the nervous system. The nutritional components are efficiently taken up by diverse cell types. The skin and mucous membranes of the human body can absorb the formulation solution over a prolonged period and across a large surface area, ensuring a safe, non-invasive, and comfortable experience.

The active absorption of the formulation solution by the human nervous system mimics the extensive and sustained uptake of various small nutrient molecules by plant roots.

Wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration.

Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair. This process stimulates the regeneration of various tissues, including the skin, muscles, nerves, blood vessels, and bones. Additionally.

Wherein said formulation solution effectively disrupts pain signal transmission and expeditiously eliminates excessive inflammatory mucus, while concurrently suppressing inflammation and tumors, resulting in pain relief. It also impedes the formation of scars and keloids, as well as the proliferation of viral and bacterial pathogens. The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair. Furthermore, it demonstrates inhibitory effects on conditions such as periodontitis, hypertension, diabetes, as well as the overall age-related decline in bodily functions, either individually or in various combinations.

. The system of claim 1, Wherein said formulation solution possesses strong lipophilic properties , enabling efficient decomposition of polycyclic aromatic hydrocarbons such as benzo[a]pyrene and disruption of the viscosity of heavy oil fouling.

Wherein said formulation solution is applied topically to various human organs, including the skin, eyes, mucous membranes, oral cavity, intestines, and hair. The human skin, mucous membranes, eyes, and lung membranes exhibit rapid absorption of the formulation solution, especially when actively absorbed by the nervous system. The nutritional components are efficiently taken up by diverse cell types. The skin and mucous membranes of the human body can absorb the formulation solution over a prolonged period and across a large surface area, ensuring a safe, non-invasive, and comfortable experience.

The active absorption of the formulation solution by the human nervous system mimics the extensive and sustained uptake of various small nutrient molecules by plant roots.

. The system of claim 1, Wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration.

Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair. This process stimulates the regeneration of various tissues, including the skin, muscles, nerves, blood vessels, and bones. Additionally.

. The system of claim 1, Wherein said formulation solution effectively disrupts pain signal transmission and expeditiously eliminates excessive inflammatory mucus, while concurrently suppressing inflammation and tumors, resulting in pain relief. It also impedes the formation of scars and keloids, as well as the proliferation of viral and bacterial pathogens. The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair. Furthermore, it demonstrates inhibitory effects on conditions such as periodontitis, hypertension, diabetes, as well as the overall age-related decline in bodily functions, either individually or in various combinations.

. The system of claim 1, Wherein said In practical applications, the formulation of the present invention is administered to any part of the human body for disease prevention or treatment. It can also be utilized for disease prevention or treatment in mammals and pets. The formulation of the present invention can be manufactured in various forms, including tablets, injections, nebulizers, ointments, patches, topical applications, acupoint patches, aerosol sprays, bottled liquids, capsules, and any other pharmaceutical production and manufacturing processes or their combinations.

### Specific implementation methods

To clarify and make the purpose, technical solution, and advantages of the present invention more comprehensible, the following embodiments provide further detailed explanations of the present invention. It should be noted that the specific embodiments described here are intended solely for the purpose of explaining the present invention and are not intended to limit the scope of the present invention.

The formula liquid described in this application is readily absorbed by the human nervous system, and contains a saturated concentration of natural small molecules such as tea has over 20 amino acids. These molecules bind to corresponding receptors on cells and transmit bioelectric signals. The liquid can be delivered to the nervous system for extended periods of time, and at fixed times and positions, it secretes mucus, delivering bioelectric signals through established nerve pathways. This results in regular mucus secretion, pain suppression, bioelectric signal delivery, and regeneration ability.

Mucus secretion is closely associated with many physiological activities and diseases in the human body. However, due to the lack of appropriate medications that can stably control mucus secretion, there has been insufficient research on the regularity of mucus secretion in humans. The complexity of the systems involved in mucus secretion in the human body makes it challenging to conduct experiments and requires a significant amount of work, making it difficult to evaluate the results.

The research team discovered that a combination solution of X, 26.47% sodium chloride, and high concentrations of tea components can be quickly absorbed by the nervous system when administered to various organs and tissues of the human body, such as through eye drops, nasal drops, eye nebulization, lung nebulization, and rectal injection. The solution is delivered in a stable manner through the nervous system for extended periods of time via electrical signals, and mucus is discharged at a fixed location after approximately 1 minute (for drops) or 30 minutes (for nebulization). From 2013 to 2021, Xu Haohan conducted a significant number of personally involved human experiments in the lungs, nose, eyes, mouth, skin, and rectum, recording and summarizing the consistent characteristics and discovering new patterns.

### Experimental Design:

Between 2012 and 2013, a lot of basic experiments were conducted on haze. The experiment produces regular results. 30 minutes the pungent smell is gone. The smoke from burning papper was mixed with the fog of atomizer atomizes the X solution and waiting for 30 minutes to remove harmful substances such as benzo[a]pyrene and accelerate smoke settling. In 2013, due to a sudden onset of acute pneumonia in the middle of the night, with severe headache and severe coughing, the fog of atomized solution X was directly inhaled into the lungs under dangerous circumstances. After 30 minutes, a large amount of mucus was coughed up, and the headache was significantly reduced, and the cough disappeared. Consequently, X was closely monitored. From 2013 to 2016, hundreds of tests were conducted on atomized lung, nasal drip, and atomized eye . It was found that X had a fixed time for mucus secretion and was closely related to the nervous system. A large number of systemic experiments are planned.

The experimental design considered the fixed neural absorption pathway, timing and location of mucus secretion, pain inhibition time and process, neural pathway, pain effect, and internal mucus secretion in the human body. Since it is challenging to observe these accurately in animal experiments, after an objective analysis, it was decided that the research and development personnel would conduct a full-body experiment on themselves to ensure complete safety. They then identified the fixed characteristics and patterns. If the results are positive, further testing of the fixed characteristics and patterns can be carried out among friends and family.

The animal experiments in this implementation example were conducted in early 2020 when the human experiments were nearing completion. The objective was to determine if X had similar fixed characteristics and patterns in animals as observed in humans. It was found that X had partially similar effects on both humans and animals.

Between 2016 and 2021, a comprehensive range of human experiments were conducted, including over 500+ eye misting, 500+ eye drop, 500+ nasal mucosa, 1000+ lung misting, 1000+ full-body application, 1000 +oral mucosa and gingival, 1000+ rectal mucosa, 500+ scalp soaking, 500 +scalp and skin microinjection, 100+ headache inhibition, and 300+ experiments involving splitting or adding X drug components.

The figure of 500+ is a conservative estimate. For instance, in the case of eye drop experiments conducted from 2016 to 2023, over 10 people were involved in the testing and performed close to 10,000 eye drops. Similarly, there were tens of thousands of experiments conducted for intestinal unblocking and oral experiments.

Based on the industrial applicability analysis, it was found that the concentration of sodium chloride in X is approximately 26.47% when it reaches saturation level. At this concentration, the solubility is around 36 (varies with temperature). The use of saturated sodium chloride in the formula solution provides stability and significantly prolongs the efficacy and disease-inhibiting ability of the small amino acid molecule components in X solution, despite the short half-life of amino acids. The extraction method for the tea water component involves high-temperature boiling and high-concentration extraction of the effective components from tea.

In the experiments, the high-dose and high-concentration tea components, including Tieguanyin tea and the mixture of high-concentration tieguanyin and sodium chloride are collectively referred to as the formula solution. For one experimental example of the formula solution, we used tea solution with a concentration as close to saturation level as possible, typically at a concentration of 40-70mg/ml or higher. The concentration of sodium chloride at saturation level is approximately 26.47%, with a solubility of around 36 (varies with temperature), and is referred to as X solution. We conducted the experiments on X solution in the following sequence:
The fundamental experiment for removing benzo[a]pyrene involves the removal of this carcinogenic substance produced by combustion. This experiment is crucial due to the harmful effects of benzo[a]pyrene.

To fill up the entire space of 12 cubic meters, X solution was atomized and turned into mist. After thoroughly mixing X mist with smoke particles from combustion, the combustion byproducts, including benzo[a]pyrene, were effectively removed within 20 minutes.

Clearing benzo[a]pyrene or clearing free radicals in the body share similarities. Free radicals are the byproduct of mitochondrial combustion in the human body and can cause significant harm by producing substances that induce inflammation, such as inflammatory products in brain neurons. This can negatively impact brain health. Timely clearance of free radicals is beneficial for one's health.

The facial oil cleansing experiment involves applying X solution and soap solution separately to the corresponding areas of the face where oil secretion is high. The solutions are then absorbed and wiped using dry tissue paper, with X solution proving to be effective in cleaning the area in 4-7 seconds. Repeating the application of X solution and absorbing it with soft paper efficiently cleanses the facial skin oil, making it a dry cleaning method for the face. This method has the advantage of simultaneously removing oil and suppressing inflammation. However, soap cleansing takes more than ten seconds and wiping it with a soft tissue paper does not provide a thorough cleaning. Soap cleansing is better suited for rinsing with water to achieve a cleaning effect.

Experiment:Researchers conducted a ten-day dry cleaning of the face using X solution. They applied the solution to the face and then absorbed it using soft tissue paper, which quickly removed oil and inflammation substances from the face. The face felt clean and comfortable to the touch after cleaning. The advantages of this method are that it reduces oil secretion, provides better sun protection and insect repellent effects, improves the skin's ability to suppress inflammation, and reduces pore size. After using X solution to clean the facial skin, it can be rinsed with water. Dry cleaning before wet cleaning can further suppress inflammation and effectively remove oil from deeper skin layers.

The statement emphasizes the fast oil-dispersing ability and unique oil-attracting properties of X solution. with soft toilet papers is appropriate for absorbing the solution, highlighting the hydrophobic and oil-attracting nature of X solution. However, it should only be used for dry cleaning, as wet washing with water would immediately remove the hydrophobic and oil-attracting properties of X solution. It is worth noting that the hydrophobic and oil-attracting properties of X solution differ significantly from those of soap and laundry detergents.

X solution offers advantages in terms of its ability to rapidly disperse and decompose oil, as well as its biodegradability. These advantages prove beneficial in removing mucus and decaying human matter in the present application, as well as clearing inflammatory infections within the human body.

In the heavy oil dissolving experiment, equal amounts of highly viscous heavy oil stains from kitchen range hoods were placed in three transparent containers. Subsequently, X solution, soap solution, and laundry detergent solution were added to each container, and all were poured in simultaneously.

Within just 2 minutes of application, X solution was able to cause 70-80% of the heavy oil stains to float to the surface of the liquid, while soap solution and laundry detergent solution only managed to make less than 5% of the oil stains float to the surface after half an hour. It is evident that X solution has a much faster rate of decomposing heavy oil stains compared to soap and laundry detergent.

The strong hydrophobicity and lipophilicity of X solution are responsible for its ability to decompose heavy oil stains. Similarly, soap and detergent solutions can prevent the growth of bacteria and viruses due to their hydrophobic properties, and X solution, with its strong hydrophobicity, can also achieve the same results. In the current implementation of this application, the strong hydrophobicity of X solution has a high efficiency in draining mucus and inflammatory products, as well as decomposing and dispersing inflammatory substances. In terms of clearing infected tissues, X solution has a significant speed advantage and excellent efficacy, which provides a foundation for achieving human regenerative capabilities.

Mouse Experiment: The experiment was conducted in the year 2000, using hamsters as the experimental subjects.

The experiment involved fog of atomized X solution from top to bottom in a semi-closed, transparent container (20cm in diameter and 20cm in height) with enough volume to fill the container, for one hour daily, for a period of 30 days. The mice displayed no signs of abnormal reactions or stimulation to the X mist exposure, and no adverse effects were observed during the study. A comparison between the mice that were sprayed with X mist and those that were not showed that the former excreted more feces and displayed greater agility.

This experiment involved testing the ability of X solution to inhibit drug-resistant bacteria found in outdoor riverbanks and soil, with multiple sampling conducted. The mice were subcutaneously injected with approximately 0.1 milliliters of X solution every three days for a period of one month, totaling over forty injections among four mice. The objective was to validate the X solution's efficacy in inhibiting bacterial and viral infections.

Several 30-milliliter glass vials were prepared,add solution X to the bottle, each containing small amounts of soil from different locations (such as riverbank mud, roadside soil, sputum-contaminated soil, park grass undersoil, tree bark, and feces). These mud were then immersed in X solution for two hours, and a small amount (less than 0.1 milliliters) of the resulting solution was extracted and subcutaneously injected into mice. After observation, the mice showed no signs of inflammation, swelling, or infection.

Analysis: X solution has a saturation concentration of 26.47% and contains high concentrations of tea and sodium chloride components, which have strong hydrophobic properties. Due to this, bacterial and viral infections present in soil are unable to propagate in X solution. As a result, subcutaneous injection of a small volume (0.1 milliliters) of X solution into hamsters did not cause any inflammatory reactions.

Small mouse experiment to clear the lungs: Two pounds of flour were placed in a transparent container and blown with an air pump to keep the flour dust suspended in the air. Mice were then placed in the container, and after two hours, they began to experience convulsions and tremors all over their body due to inhalation of the flour dust, similar to the symptoms of Parkinson's disease patients. They became extremely weak and unable to move, showing critical danger to their life.

After transferring the mice to a transparent container of equal size, they were atomized with fog of X solution. Within 5 minutes, the mice started using their paws to clean their mouth and nasal cavity. After 20 minutes, their convulsions and tremors stopped, and they returned to normal activity. One hour later, the mice had fully recovered and were agile in their movements and responses.

Experimental analysis: The mice in the experiment suffered from respiratory distress and airway blockages, which caused difficulty breathing and hindered oxygen delivery, resulting in involuntary tremors similar to those seen in Parkinson's disease in hamsters. The mice were then transferred to another nebulization container and exposed to a large amount of fog, which they inhaled into their lungs.

The X solution was nebulized into the lungs of the animals for a fixed duration, and it did not cause any painful or irritating reactions. The mice were well-tolerated to the nebulized X solution and the results were similar to those observed in humans.

Tea offers a safe and effective alternative for the clinical treatment of lethal bacterial and viral pneumonia. Wu yonglin Tianjin University Researchers investigated the antibacterial mechanism of tea against methicillin-resistant Staphylococcus aureus (MRSA), including its target amino acids and transmembrane interactions. In pig experiments, it demonstrated excellent biological safety. In a mouse model of lethal H1N1-MESA mixed pneumonia, nebulized inhalation therapy with tea showed promising treatment efficacy due to its rapid clearance of H1N1-MESA and antioxidant interactions. The small tea molecules heavily aggregated on the surface of MESA bacteria, inducing MRSA membrane permeation, disrupting the entire membrane structure, and ultimately killing the bacteria.

In 2020, Zhejiang Provincial Center for Disease Control and Prevention selected green tea, Tieguanyin and Dahongpao, two grams each, soaked for 30 minutes with 50 ml of boiling water (measured at 90 degrees), at this time the concentration meter is 40mg/ml (usually the first tea when making tea), cooled to room temperature, diluted to each experimental concentration with cell maintenance solution. In vitro virus elimination experiment was carried out with tea water of different concentrations (2.5-10mg/ml). Vero cells were infected and cultured for 48 hours after tea water of different concentrations and the control group were treated with 100tcid50 novel coronavirus for 1 hour, and the proliferation level of viral nucleic acid in each group was measured. The results showed that compared with the control group without tea treatment, the nucleic acid proliferation of the novel coronavirus in the tea treatment group was reduced by 10,000 to 100,000 times, and the virus inactivation was close to 100%.

However, it should be noted that the tea component does not possess neuroabsorption function, fixed-time mucus secretion function, or the ability to block pain signals.

During the mouse experiment, no inflammation was observed, and the airways remained patent. The safety profile was excellent, and the mice exhibited calm behavior without any pain response. The treatment efficacy observed in mouse experiments was comparable to that seen in human experiments.

### Human safety experiment:

The sodium chloride in X solution is present at a saturation concentration, and the tea component approaches its highest solubility for safe use in the human body.

For instance, if 300g of tea is boiled in 2 kilograms of water for ten minutes, soaked for fifteen minutes, and then poured into a glass or porcelain container and mixed with sodium chloride, the resulting solution will be X solution.

The human experiment with X solution demonstrated regular mucus secretion, pain suppression, and inflammation control at a fixed time and location, owing to its close association with the nervous system.

The experiment soaking using X solution, or the experiment atomized X solution produced stable and consistent results every time, with similar effects observed at a fixed time.

The implementation of rectal injection experiment involves rectal clearance once or twice a day, which is an essential process for supplementing neurotransmitters in the elderly. This experiment has been conducted thousands of times. X solution is injected into the rectum from the anus in a volume of 4-10 milliliters for about a minute, which accelerates the reflex of intestinal defecation, initiates gas discharge, urination, and smooth defecation. Daily use of X solution can quickly alleviate inflammation and pain caused by hemorrhoids, anal fissures, and colon cancer. Additionally, the rectum secretes mucus during the process. The use of X solution for defecation does not cause anal fissures because the mucous secretion and absorption of X by the nervous system adequately lubricate the intestinal tract, eliminating the risk of straining during defecation and increase in blood pressure. Therefore, the defecation process becomes comfortable.

During the implementation of pulmonary nebulization experiments, the X solution was nebulized for about 30 minutes, with 10-20 milliliters of the solution being used for thousands of experiments. The nebulization process involved inhaling through the mouth and exhaling through the nose to allow the X mist to penetrate deep into the lungs. Most participants started coughing and expectorating after around 7 minutes of nebulization, as the pulmonary sensory receptors (such as cough receptors) and immune system worked together to expel mucus, transporting it to the throat and actively coughing it out. After half an hour of nebulization, 40-60 milliliters of mucus could be expelled. Nebulization can be performed multiple times a day to expel inflammatory substances and fluid accumulation in the lungs.

An elderly woman in her 70s experienced severe and unexplained right-sided lower back pain after contracting pneumonia. However, the pain was quickly alleviated after nebulizing the lungs. Another elderly patient had asthma, tuberculosis, a hunched back, dizziness while walking, and severe tremors due to Parkinson's disease. The patient underwent nebulization of the lungs twice a day using X solution, alongside nebulizing the eyes and using eye drops. During the six-month period, the patient did not take any other medication and underwent a medical examination, which revealed that the lungs were healthy without any abnormalities. The patient also experienced a significant improvement in physical strength, and the tremors due to Parkinson's disease were practically eliminated by soaking the nipples in the solution for an hour daily, for two weeks.

### injection experiment:

The research and development personnel had been experiencing baldness on the top of their head for twelve years and had maintained a flat hairstyle. They did not feel any pain when their scalp was punctured. They received a micro-needle injection of a formulated solution, with each injection at 0.01 milliliters, at over twenty spots on the top of their head, once a week for four consecutive weeks. After the scalp was punctured, they felt some pain, and hair started to grow on the top of their head. It was speculated that the disappearance of nerves near the scalp hair follicles led to hair loss. However, after the micro-needle injection, the nerve receptors around the hair follicles on the scalp were significantly repaired and regenerated, leading to natural hair regrowth. It was observed that using the formulated solution daily, which followed a regular sodium ion spiral curve, promoted the repair and regeneration of damaged hair follicle nerves.

As per a Cell journal article, researchers from Harvard University have found that there is a link between the sympathetic nerves present in the skin and small smooth muscles in the stroma. Additionally, there exists a neuromuscular junction between the sympathetic nerves and muscles, and a connection between the sympathetic nerves and hair follicle growth cells. When degenerated sympathetic nerves are repaired successfully, hair follicles can grow hair, indicating a direct relationship between the repair of the nerve system and hair regeneration.

An elderly relative of mine suffered from a lumbar disc herniation that caused immense pain, rendering them unable to walk. The condition also caused them to lose weight, dropping from 50 kg to 40 kg. To alleviate the pain, they received four micro-needle injections of 0.01 milliliters of a specially formulated solution at the site of the pain, leading to immediate relief. The elderly person used the same solution to clear their bowel daily and received an injection of the same solution every two days. After a month, they could walk freely again, and their weight returned to 50 kg. A hospital follow-up a year later revealed that the lumbar disc herniation had transformed into intervertebral dies bulge. The elderly person continues to maintain a daily routine of clearing their bowel and receives a weekly micro-needle injection of the formulated solution. It was observed that using the solution daily, which followed a regular sodium ion spiral curve, led to partial repair and regeneration of lumbar inflammation and lumbar injury through skin needle injection.

The research conducted personal experiments on the effectiveness of injections on various body parts, including the toes, knee joints, sciatic nerves, lumbar vertebrae, cervical lymph nodes, and scalp. They administered subcutaneous injections on the body hundreds of times, injecting 0.1 milliliters each time. The injected solution formed small droplets that disappeared quickly, which were absorbed by the nervous system. After the solution was delivered and absorbed, it rapidly biodegraded and vanished from the body, without causing any inflammatory reactions.

### gum and teeth experiment:

The experiment involved pouring about 20 milliliters of a solution (referred to as X) into the mouth once a day. Upon the first use, the participant experienced slight toothache and stimulation, with signals observed along the teeth and alveolar bone, along the dental nerve, and diffused to the entire alveolar ridge, followed by numbness. However, after one minute, the pain subsided, and after several days of continuous use, tooth sensitivity was significantly reduced and enamel damage was automatically repaired.

After using the solution every day for one minute, the alveolar ridge and oral cavity became numb. Over the course of one to four weeks, the gums began to show signs of muscular growth, the teeth became tighter, tooth sensitivity was reduced, and tartar was reduced. Continuing to use the solution 1-2 times a day for more than a year, the gum muscles formed a tight ring around the teeth, and common problems like tooth sensitivity, enamel loss, and inflammation were eliminated.

Pour approximately 20 milliliters of X solution into the mouth once a day. Initially, there may be slight toothache and stimulation, and signals may be observed along the tooth seam to the alveolar bone, spreading along the dental nerve to the entire gum, followed by numbness. After 1 minute, the pain subsides. After several days of continuous use, tooth sensitivity significantly reduces, and enamel damage is automatically repaired. One minute after using the solution every day, the oral cavity becomes numb. From one to four weeks later, the growth of gum muscles becomes very noticeable, teeth become firmer, tooth sensitivity decreases, and dental calculus decreases. Continuing to use 1-2 times a day for more than 1 year, the circular muscle of the gums firmly secures the teeth, and common problems such as tooth sensitivity, enamel loss, and nerve stimulation caused by tooth inflammation disappear.

An Example of Pain Inhibition Experiment: X solution has been shown to inhibit pain caused by common muscle soreness, skin inflammation, mosquito bites, skin bruises, and burns. Soaking or applying a large area is effective, with severe pain being quickly relieved in just 1 minute of soaking. Soaking for more than 20 minutes significantly reduces or eliminates skin and muscle pain.

Injuries resulting from exercise can cause persistent numbness or tingling due to ongoing nerve transmission problems that may not respond well to topical applications due to internal inflammatory problems within the nervous system. In such cases, soaking the mucous membranes of the nipples, genitals, or using pulmonary nebulization for more than 30 minutes can be effective. For instance, joint pain, especially muscle soreness that occurs on rainy days, can be relieved by soaking the mucous membranes. Mild pain can be restored to normal within 4-7 days of soaking, and persistent soaking can gradually reduce symptoms of more serious conditions such as bone inflammation and improve inflammation clearance.

An elderly individual was suffering from severe hunchback, with significant arm pain occurring whenever they laid their head flat. By soaking their nipples in X solution for four to five hours each day over a period of three weeks, the individual was able to lay their head flat, and their arm pain reduced to 90%. Furthermore, the individual's hand tremors caused by Parkinson's disease disappeared. Most importantly, the individual's height increased by 1.5 cm, and their hunchback is gradually becoming less severe.

The researchers had been suffering from cluster headaches for more than ten years and were diagnosed with vascular stenosis in the hospital. At its worst, they experienced headaches several times a week, including long-lasting, bell-ringing headaches that kept them awake all night. However, after using eye drops daily for a month, the cluster headaches disappeared, and they have not experienced any symptoms of cluster headaches for many years.

### Eye Experiment:

The solution's formula quickly breaks down inflammatory components (including in the brain) in any part of the body, and then excretes them from the body after lymphatic exchange.

The X solution is atomized into the eyes for about 30 minutes. There have been two to three thousand tests on participants of all ages, including the elderly and middle-aged. The sensation of atomizing X solution into the eyes is cool and painless. The purpose of atomization is to suppress headaches caused by colds or unknown causes, and to improve vision by suppress inflammation. After 1 to 8 weeks of X atomization, macular holes and metamorphopsia significantly improved, floaters decreased, and inflammation and visual acuity significantly improved.

There is a notable difference in the effect between X solution eye drops and atomization. After approximately 1 minute of eye drops, mucus is discharged from a fixed position on the throat wall. This discharge reduces and eliminates pain significantly because the lymphatic vessels around the eyes and meningeal lymphatic vessels are interconnected. Eye drops produce a higher concentration of neurotransmitters behind the cornea of the upper and lower eyelids, and the point-to-point electric signal delivers the neurotransmitter to the occipital bone position, prompting the meningeal lymphatic vessels to discharge inflammatory mucus. This mucus then reaches the lymphatic vessels and is discharged from the throat wall. Sodium and chlorine ions are at saturation concentration and bind to corresponding receptors with multiple amino acids and cells, triggering different signal pathways and blocking and suppressing pain signals. The secretion of mucus through eye drops is the result of the high-speed linkage between the central nervous system and the immune system. Eye drop tests on different people show the same secretion time, position, and amount of mucus. Severe brain inflammation results in more mucus discharge after eye drops, while less mucus is discharged after eye drops when brain inflammation is reduced.

Eye drops have a faster inhibitory effect on eye diseases compared to atomization.

The fixed path of the eye drop experiment is from the eyes to the occipital bone, then to the sacrum, and finally to the heart.

Characteristics: discharge of mucus, pain inhibition; eye drops are more effective when applied to the lower and upper eyelids, easing eye pain, headaches, and spinal pain; eye drops accelerate the discharge of mucus from the meningeal lymphatic vessels; some mucus is discharged from the position near the posterior pharyngeal wall and the occipital sinus. The amount of mucus discharged is more when there is more inflammation, and less when there is less inflammation.

Observation should be done once or twice a day. When applying eye drops, use your finger to pull down the lower eyelid and apply drops continuously for one to two minutes. Then, use your finger to pull up the upper eyelid and apply drops continuously for one to two minutes. Inflammatory substances in the eyes will be discharged after applying eye drops. Participants in the experiment may feel pain at the beginning, but the pain gradually disappears after one minute. After applying eye drops, the heart rate increases for a short period but returns to normal soon after. Eye drops provide fast relief for eye and brain pain. They also help to alleviate brain swelling and accelerate the discharge of inflammatory substances from the brain. Continuous use of eye drops can promote the growth of eye muscles, significantly improve the orbicularis oculi muscle and temporal muscles, and reduce the appearance of wrinkles around the eyes. The use of eye drops can also significantly improve heart function, reduce heart rate after exercise, and enhance exercise capacity.

The researchers observed that after applying eye drops, mucus was discharged near the occipital sinus at the back of the pharyngeal wall, leading to the instant relief or disappearance of brain inflammation and pain. During the eye drop experiment, the researchers dropped about 0.02 milliliters of the formula solution at the lower and upper eyelid positions, applying eye drops every half an hour, more than 20 times in 24 hours. This resulted in the enhancement of electrical signals and a significant feeling of an instant transmission of electrical signals from the eyes to the occipital bone, from the occipital bone to the sacrum, and then to the heart through a point-to-point jumping method. It takes only about 0.6 seconds to transmit electrical signals from the eyes to the heart in this way, and this method can last for more than 24 hours.

The researchers also found that continuous application of eye drops for more than 20 times per day, and continuously for seven days, can cause individuals to feel a continuous jumping pattern from the eyes to the occipital bone, from the occipital bone to the sacrum, and from the sacrum to the heart when their eyes are closed. The continuous strong signals have a significant impact on sleep, causing fatigue, weakness, and muscle soreness, but no other discomforts. Stopping eye drops for three to four days can quickly restore normal conditions. The researchers speculate that the concentration of sodium ions absorbed by the nervous system increases, while the rate of metabolism of sodium ions is relatively slower than absorption, leading to the accumulation of an excessive amount of sodium ions in the nervous system. This can be restored to normal after stopping eye drops for three to four days, allowing the excess sodium ions to be excreted from the body. Case study:
Between 2008 and 2012, a researcher experienced headaches caused by abnormal brain blood vessels. The frequency of headaches increased from one or two times a month to one or two times a week, accompanied by a feeling of heaviness in the head while walking. Hospital examinations revealed lacunar infarction with two cavities in the brain. Intravenous injection at the hospital worsened the headache, leading the researcher to rest at home. The researcher then attempted fasting and limiting their diet, which provided some relief and reduced the frequency of headaches to one or two times a month. In 2016, the researcher began using a formula for eye drops and noticed that daily use of the drops significantly reduced their headache symptoms. They no longer needed to limit their diet, and their sleep returned to normal. A new brain CT scan showed that the lacunar infarction in both the left and right brain had disappeared, and the cavities in the brain were repaired. After using the eye drops for many years, the researcher's heart function improved significantly, and their endurance increased. The researchers observed that daily use of the formula solution resulted in a regular spiral curve of sodium ions, which repaired and regenerated brain inflammation and injury.

In another case, a family member of the researcher was hospitalized for subarachnoid hemorrhage with brain ventricle rupture, brain edema, and respiratory failure. The researcher observed the patient's eyes and found that the iris and sclera were blurred with severe blood clots and bubbles. The researcher used the formula solution for eye drops, applying 0.04 milliliters to the lower eyelid for about two minutes each time. After 24 hours, a large amount of inflammatory protein discharged from the upper and lower eyelids. The researcher applied the eye drops about ten times a day and observed that there was a significant discharge of inflammatory proteins from the eyes daily. After continuous use of the eye drops for ten days, the patient's sclera and iris became brighter, and the brain edema disappeared.

The subject of the study had presbyopia, eye inflammation, and multiple blood vessels in their eyes. They used X solution formula for eye drops as a mist for 30 minutes once a day for 15 consecutive days. As a result, their eyesight improved, the inflammation in their eyes was suppressed, and the number of blood vessels decreased significantly.

In another case, the subject suffered from eye inflammation and blurry vision, making it difficult to see clearly. They used X solution formula for eye drops as a mist for 30 minutes once a day for seven consecutive days. As a result, their eyesight significantly improved, and their vision gradually became clearer.

A relative of the researcher was suffering from a degenerative eye disease, which included retinal detachment, cloudy vitreous, and macular hole, causing severe distortion in their vision, and requiring surgery. The patient voluntarily participated in an eye drop experiment and did not use any other medication during the study. After three months of using the eye drops, it was observed that the distortion in their vision disappeared, and their eyesight improved significantly. One year later, an eye examination revealed a clear vitreous, a normal retina, and an improved macular hole. It was noted that the daily use of the formula solution resulted in a regular spiral curve of sodium ions, which repaired and regenerated eye inflammation and injuries.

Misting the eyes with X solution is a very conservative treatment that can quickly reduce the patient's discomfort.

Misting the eyes with X solution has been shown to significantly improve the quality of sleep and alleviate symptoms of depression.

The study participant, who was about 50 years old, had been experiencing interrupted sleep and waking up at 2 am for almost six months, unable to fall back asleep until morning. However, after misting their eyes with X solution for 30 minutes each day, they noticed some improvement in their insomnia after one day. Their sleep quality improved significantly, and they were able to sleep more soundly until 5 am. After misting for ten consecutive days, their sleep continued to improve, and they were able to sleep until 7 or even 8 am. The researchers observed that the increase in neurotransmitters in the body after the absorption of the X formula's ingredients was directly related to the improvement of sleep quality.

X solution has been found to have an inhibitory effect on Parkinson's disease. For elderly people experiencing hand tremors, both misting the eyes and dropping X solution into them can quickly suppress tremors. Consistent misting of the lungs and eyes every day can effectively suppress hand tremors, and soaking the nipples in X solution for over half an hour each day can also be effective in inhibiting Parkinson's disease, with visible results within a month. Overall, dropping X solution into the eyes appears to be more effective than misting, as it requires less time and achieves faster results.

An experiment was conducted to test the effects of X solution on muscle growth:
Regular application of the solution to a large area of the body for 20-40 minutes each day helps to gradually rid the tissue under the skin of harmful substances, restore nerve endings and capillary networks, and promote the growth of skeletal muscles. Continued use of X solution leads to rapid growth of finger muscles, a significant increase in grip strength, and visible growth in chest, arm, and thigh muscles. This is a convenient way to maintain strong muscles, even for those who are not particularly active.

In addition, soaking the nipples and genitals in X solution helps to reduce inflammation in the body's nervous system and promote muscle growth.

Researchers also applied drops of X solution to their eyes twice a day for over four weeks, which gradually thickened the eye and temporal muscles. Combining nose drops with eye drops for more than eight weeks resulted in significant growth of cheek muscles, eye and temporal muscles, and reduced wrinkles. Long-term daily use of eye drops (1-2 times a day) can help maintain muscle strength around the eyes and prevent them sinking toward the eye sockets. The decline of the eye sockets in the elderly is usually related to the degeneration of the muscles around the eyes. Using nose and eye drops twice a day can inhibit the degeneration of these muscles and prevent the decline of the eye sockets. Researchers recommend this method for family and friends. After eight weeks of using nose and eye drops, a 70-year-old person's cheek and eye muscles showed a significant increase.

This is a method to repair nerves and muscles for patients with muscle atrophy, and it is also a way for the elderly to maintain muscle mass and prevent muscle degeneration.

The X solution is capable of delivering a considerable amount of beneficial ingredients into the synapses. This leads to the process of endocytosis in synaptic vesicles, where the human body synthesizes a wide variety of neurotransmitters that can promote a positive mood, inhibit negative emotions, and improve lung health by aerosolizing into the lungs. As a result, the oxygen supply is improved, and emotional well-being is enhanced. The use of X solution can also help alleviate symptoms of depression.

The X solution improves the diversity of neurotransmitters and eliminates infectious and inflammatory substances, leading to a significant enhancement in sleep quality. In experiments, the use of X solution eye drops has shown to improve sleep quality and alleviate some unexplained headaches. Consistent use of X solution nose drops promotes the secretion of fixed mucus, which reduces inflammation in the nasal cavity and upper respiratory tract. This may alleviate or improve snoring, resulting in a noticeable improvement in sleep quality.

Inflammatory experiment of X solution: During the experiment, cuts, scratches, or other injuries on the hands or legs were immersed or coated with X solution. The solution showed a significant ability to suppress inflammation and reduce swelling. In everyday life, it is convenient to treat various injuries such as cuts and scratches caused by marine fish. After continuous coating and soaking with the solution, the wound does not become inflamed or swollen, and pain is quickly alleviated, promoting self-healing.

### AnExample experiment on muscle, blood vessels, and skin regeneration:

Regenerative medicine refers to the process of accelerating the body's self-repair mechanisms to a level that is clinically significant.

### Case study of skin, muscle, nerve, and blood vessel regeneration:

A patient suffered a 1.5 cm long wound on their calf muscle caused by a sharp construction tile, which was left unsutured. After two months of treatment with iodine, phenol, and alcohol, the wound became infected with drug-resistant bacteria, resulting in an oval-shaped small pit that corroded the skin and muscle. The outer rim of the pit was raised and hardened, and the muscle was covered with pus and necrotic tissue. The pus and tendon muscles were tightly adhered, with a depth of 0.5-0.8 cm, and the open wound infection area was 2.5 cm x 1.5 cm. Pus was clearly visible in the skin around the infection, and the wound continued to grow while emitting an unpleasant odor.

After multiple cycles of soaking the wound with X solution for 20 minutes each time, the pus material was easily removed, and the adhesion between the pus and tendon muscles was cleared. The X solution penetrated the infected area, and the process caused minimal pain. Six hours later, a blood scab formed, and after four days, a thin, transparent scab formed around the blood scab, which was about 3 mm wide. Then, a syrup-colored liquid flowed out from different positions under the transparent scab every day. After solidifying, the scab slowly thickened, resembling a 3D print, and continued to increase in height and thickness under the transparent scab. The thickest part of the scab reached a thickness of 0.4-0.5 cm.

After a duration of four weeks, the patient accidentally removed the scab, which revealed that the skin and muscle had regenerated two-thirds under the scab without any scarring. A shallow pit of approximately 1.5 square centimeters was visible under the scab, containing floating fat, stem cells, and repair fluids. The fat floated on the liquid resembling a lotus leaf, and the entire repair process was painless. After the scab fell off, the wound was again soaked with X solution, and a new scab formed six hours later. Four days later, a small scab appeared in the center, which was surrounded by a thin, transparent scab, constituting one-fourth of the previous size. The edge of the small scab continued to thicken, and the process was repeated until the scab fell off. The skin and muscle were entirely regenerated without any scarring, and the regeneration process followed a strict and precise procedure.

During the process, the tendons in the injured leg might feel slightly uncomfortable, indicating the need to apply X solution to the affected area. It is crucial to apply the X solution around the scab and not directly onto the scab to prevent its breakdown and destruction. Therefore, it is essential to apply the X solution around the scab daily to ensure proper healing.

The regenerated skin has distinct skin texture.

### An example of melanoma removal:

The experimenter had a melanoma of 2 square centimeters on the scalp, above the ears, which originated as a small mole and gradually developed into an irregular surface lump. They used a microneedle to inject 0.1 ml of X solution into the melanoma surface twice. Afterwards, they picked and broke the skin of the lump and applied X solution to the melanoma for conservative treatment by soaking and topical application.

The melanoma continuously scabbed, shrunk, scabbed, and shrunk.

After two months, the melanoma disappeared completely without any scarring, and there were no visible scars on the face.

### AnExample of knee pain:

The experimenter had knee joint pain and could not walk for more than an hour without severe pain due to a cracked meniscus.

They applied X solution to the knee joint daily, whenever they felt discomfort, 4-10 times a day. They also applied it to the thigh and calf muscles at night to aid in the treatment. After 10-30 days, the knee pain anesis, and they were able to walk and run slowly.it is suggested that soaking mucous membranes is better.

Consistent application strengthens bones and muscles and repairs cartilage in the joints.

### Preliminary conclusion of regenerative medicine:

Regenerative medicine is simply speeding up the body's self-repair process to a clinically significant extent. Humans possess the ability to regenerate, but initiating the process is challenging.

Regenerative medicine can significantly shorten the treatment time for various diseases. X solution's regenerative function can accelerate the body's self-repair process. For instance, when X solution is sprayed into the eyes for 30 minutes from the onset of a headache, the pain is relieved, and after resting for 6-10 hours, the symptoms are almost completely gone, reducing the recovery time to half a day. When used as eye drops, the pain is relieved within 5 minutes, and after sleeping, the nerve pain in the head is significantly reduced.

Treating periodontitis, toothache, and loose teeth can be challenging, but when X solution is applied to the teeth and gums, 10 ml per day for 2 minutes and then spit out, and repeated for 7-15 days, the gum muscles become firm and strong, teeth become stronger, and tooth sensitivity disappears. Periodontitis is almost cured. Based on the above cases, an analysis of human experiments has been summarized. When X is applied at a fixed time and location on the human body, a fixed secretion pattern of mucus is produced. When inflammation is strong, mucus secretion is high. When inflammation disappears, mucus secretion stabilizes, and when inflammation is gone, very little mucus is secreted.

When X solution is used as eye drops, it should be kept in the lower eyelid for one minute, and then in the back of the throat for about one minute to secrete mucus. When used as nasal drops, it should be kept in the nasal cavity for about 20 seconds, and then in the nasal cavity for about one minute to secrete mucus. When X solution is sprayed into the eyes, it should be kept in the back of the throat and nasal cavity for about 30 minutes to secrete mucus. When fog into the lungs, it should be inhaled through the mouth and exhaled through the nose for about 30 minutes, and mucus secretion occurs from the throat to the oral cavity. When X solution is injected into the rectum, mucus secretion occurs for one minute. When applied to the skin, inflammation subsides after about 1 minute.

When X solution is applied to the human body, the nervous system actively absorbs it. Pain signals are blocked and suppressed for about 1 minute. After the nervous system absorbs X solution, there is a fixed signal path: the nervous system absorbs X, then secretes mucus, and almost simultaneously, pain is significantly reduced or eliminated. After about 1 minute of eye drops, mucus is secreted, and head pain is significantly reduced or eliminated. After about 1 minute of nasal drops, mucus is secreted, and nasal pain is significantly reduced or eliminated. After 1 minute of mouthwash, the mouth becomes numb, and oral pain is significantly reduced or eliminated. After 1 minute of rectal injection, mucus is secreted, and hemorrhoid pain is significantly reduced or eliminated.

Subcutaneous injection of X solution in the lumbar spine involves both muscle and skin, and therefore has a slower absorption rate by the nervous system. It takes about 10 minutes for the nervous system to absorb the solution, and significant pain reduction or disappearance is observed. Similarly, subcutaneous injection in other areas also leads to significant pain reduction or disappearance after about 10 minutes.

Research by Harvard University has shown that 20 pain receptors connect to a single lymph node. It is speculated that X solution, with its saturated sodium ions, chlorine ions, and various amino acids, quickly blocks pain signal transmission after being absorbed by the pain receptors in the nervous system. Simultaneously, mucus discharge and the collaboration between the nervous and immune systems work to eliminate inflammatory components and signals.

Repeated use of X solution as eye drops enhances its absorption by the eyes, ensuring continuous signal delivery for about 12-24 hours. Instantaneous point-to-point signal delivery is observed from the eye to the occipital bone, and then from the occipital bone to the sacrum position and from the sacrum to the heart, with a speed of approximately 1 second.

Linear transmission is used when X solution is used as nasal drops, injected into the rectum, used as mouthwash, nebulized in the lungs, or applied to the skin. For instance, the linear transmission method is used from the nasal side to the trigeminal nerve and from the nasal side to the forehead when used as nasal drops. The linear transmission method is used from the teeth to the alveolar bone during mouthwash, numbing the mucosa. Pain gradually reduces and disappears through linear transmission when nebulized in the lungs for about 10 minutes. Point-to-point instantaneous signal delivery method is always used for signal delivery from the eye to the occipital bone, from the occipital bone to the sacrum position, and from the sacrum to the heart. After eye drops, signal delivery is instantaneous through point-to-point method to the heart. This indicates that point-to-point method is simultaneously used in the eyes, occipital bone, sacrum, and heart, achieving long-distance and long-term biological signal delivery.

When X is applied to the human body, the nervous system actively absorbs the X solution formula. The absorption occurs almost simultaneously with the discharge of mucus by the immune system and the blocking and inhibiting of pain signals. This mechanism is due to the linkage between the nervous and immune systems that occurs when the formula solution is absorbed.

Extensive experiments have gradually improved and further confirmed the close association between the nervous and immune systems. The point-to-point method is bringing about youthful changes to the body. After using X solution as eye drops, electrical signals lead to youthful changes in the heart. A researcher who was 47 years old tested this on himself by using eye drops for 8 weeks. He experienced significant changes in his heart. His running speed increased, and he felt less intense heartbeat amplitude. The time for his heartbeat to return to a normal rate after stopping running also decreased. Climbing stairs became easier, and he no longer felt out of breath.

Muscle growth around the eyes was also noticeable. After 8 weeks, the levator palpebrae muscle and temporal muscle thickened and increased in height, and fine lines around the eyes decreased quickly. X solution as eye drops can significantly improve sunken eye sockets, and the growth of eye muscles can bring back the lower limit of the eyes, suppressing aging from the inside out.

In ancient China's Tang Dynasty, the scientist and doctor Sun Simiao proposed the phlegm theory, which suggests that inflammatory mucus is distributed throughout the body, causing various difficult-to-treat diseases such as brain diseases, lung diseases, tumors, skin diseases, and intestinal diseases. If the inflammatory mucus is quickly eliminated, the diseases will naturally be cured.

The human experiments with X solution have verified the theory of Sun Simiao, the doctor from the Tang Dynasty.

The ingredients in this formula are rapidly absorbed by the human nervous system, and drug delivery is achieved through long-term, continuous electrical signal delivery that blocks and inhibits pain signal transmission. Amino acid peptides are natural proteins that bind to corresponding receptors on nerve cells, sending biological electrical signals. This leads to regular secretion of mucus, regular suppression of pain, and regular biological electrical signals. These regularities are of great help to the nervous system, promoting nerve axon regeneration, repairing myelin sheath wrapping ability, strengthening neuronal signal delivery, regenerating blood vessels, reducing inflammation and scars, and avoiding physical barriers to regeneration and repair.

Eye drops accelerate optic nerve repair, and signals continue to be delivered from the eye to the brain, promoting axonal repair of neurons, repairing the information delivery pathway from the eye to the brain, and promoting cell proliferation and regeneration to form new blood vessels to deliver nutrients to the damaged area. This process of repair and regeneration is a common principle mechanism of the human brain and different tissues. The above experiments are safe, with minimal side effects, and the replication of these experiments is not complicated, making it possible to conduct them in any laboratory or clinical trial to achieve results similar to those of the present invention.

an anti-aging and regenerative formula the formula includes tea components and sodium chloride, potassium chloride, and any combination of sodium salts, potassium salts, and calcium salts permitted by any pharmaceutical. Tea has more than 500 ingredients, 500 tea ingredients, Vitamins (C, B1, B2, B3, B5, B6, B11, B12, P1, H, A, D, E, K Etc.), amino acids (theanine, aspartate, serine, glutamic acid, glycine, tryptophan, valine, histidine, arginine, threonine, alanine, proline, cysteine, tyrosine, methionine, lysine, isoleucine, leucine, phenylalanine, etc.), alkaloids (caffeine, tealeine, theobromine, etc.), tea polyphenols (catechin, flavonoids, flowers, etc.) Pigments, phenolic acids, etc.), inorganic substances (potassium, phosphorus, calcium, magnesium, iron, sulfur, aluminum, fluorine, etc.), organic acids, lipids (phospholipids, triglycerides, sulfur lipids, glycolipids), pigments (chlorophyll, anthocyanin, carotene, lutein, etc.), aromatic substances, enzymes. One of the formulation of the solution includes Tieguanyin tea, sodium chloride, and water. the tea is boiled to produce a high concentration of tea, while simultaneously achieving a state of saturation in the presence of sodium chloride (reaching the maximum saturation level of sodium chloride dissolution).

Wherein said formulation solution possesses strong lipophilic properties , enabling efficient decomposition of polycyclic aromatic hydrocarbons such as benzo[a]pyrene and disruption of the viscosity of heavy oil fouling. Wherein said formulation solution is applied topically to various human organs, including the skin, eyes, mucous membranes, oral cavity, intestines, and hair. The human skin, mucous membranes, eyes, and lung membranes exhibit rapid absorption of the formulation solution, especially when actively absorbed by the nervous system. The nutritional components are efficiently taken up by diverse cell types. The skin and mucous membranes of the human body can absorb the formulation solution over a prolonged period and across a large surface area, ensuring a safe, non-invasive, and comfortable experience.

The active absorption of the formulation solution by the human nervous system mimics the extensive and sustained uptake of various small nutrient molecules by plant roots.

Wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration.

Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair. This process stimulates the regeneration of various tissues, including the skin, muscles, nerves, blood vessels, and bones. Additionally.

Wherein said formulation solution effectively disrupts pain signal transmission and expeditiously eliminates excessive inflammatory mucus, while concurrently suppressing inflammation and tumors, resulting in pain relief. It also impedes the formation of scars and keloids, as well as the proliferation of viral and bacterial pathogens. The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair. Furthermore, it demonstrates inhibitory effects on conditions such as periodontitis, hypertension, diabetes, as well as the overall age-related decline in bodily functions, either individually or in various combinations.

Human beings possess inherent regenerative capabilities that have remained dormant. Experimental findings have unveiled a crucial correlation between the regenerative capacity of the human body and the nervous system.

Currently, there are three internationally recognized regenerative medical technologies. The first one is cell therapy, The second type of regenerative medical technology utilizes foreign materials to stimulate the body's repair mechanisms. The third type of regenerative medical technology involves the utilization of devices that alleviate the body's burden and facilitate self-healing processes.

Distinctions between this application and the forefront of international regenerative medicine: This application sets itself apart in several ways. Firstly, it eliminates the need for ex vivo cultivation or injection of stem cells, making the process more convenient and accessible. Secondly, it avoids the requirement for small intestinal mucosa, reducing the level of artificial intervention.

To tackle the challenges of aging and regeneration, the present invention offers a formula that inhibits aging and promotes repair. The formula is absorbed by the human nervous system and undergoes conversion into various neurotransmitters, generating high-level electrical signals that propagate along the established neural pathways. This allows for the route of administration through the fixed neural pathways. Simultaneously, the sodium ion concentration outside nervous cell membrane can increase from 150 mmol/l to 4524 mmol/l (the saturation concentration of sodium ions) before returning to 150 mmol/l. Through repeated daily use of the formula solution, a continuous and stable spiral curve is formed, ranging from 150 to 4524 and back to 150, enabling the regeneration and repair of human tissues and organs. During this spiral curve process, when the sodium ion concentration outside nervous cell membrane exceeds 440 mmol/l, which is observed in regenerative animals such as octopuses and squids, it triggers the regenerative capacity of humans. By continuously accumulating this regenerative capacity, it becomes possible to achieve the regeneration and repair of human tissues and organs.

Moreover, when the sodium ion concentration outside nervous cell membrane reaches 4524 mmol/l during the spiral curve process, surpassing the extracellular sodium ion concentration of malignant tumors at 451 mmol/l, the electrical signals generated at 4524 mmol/l can rapidly block pain signals caused by 451 mmol/l. This mechanism inhibits the generation of cancer-related pain. Additionally, it facilitates the rapid discharge of inflammatory mucus, suppresses the replication of viruses and bacteria, and inhibits inflammation and cancer. As a result, it effectively combats diseases associated with aging, achieving the desired effect of delaying the aging process.

This anti-aging and regenerative formula boasts minimal side effects, excellent efficacy, and a straightforward application process.

One of the formulation of the solution includes Tieguanyin tea, sodium chloride, and water. the tea is boiled to produce a high concentration of tea, while simultaneously achieving a state of saturation in the presence of sodium chloride (reaching the maximum saturation level of sodium chloride dissolution).

herein said formulation solution possesses strong lipophilic properties , enabling efficient decomposition of polycyclic aromatic hydrocarbons such as benzo[a]pyrene and disruption of the viscosity of heavy oil fouling.
it focuses on targeted application to specific areas such as the skin, mucous membranes, pulmonary mucosa, intestinal mucosa, upper and lower eyelids, and retina. The formulated solution can be administered through nebulization, topical application, or immersion.

The key mechanism of this approach lies in the active absorption through the nervous system. By absorbing the formulated solution, it effectively eliminates inflammatory mucus and disrupts or replaces pain signals, resulting in pain inhibition and facilitating tissue regeneration and repair. The benefits of this regenerative method include its non-invasiveness, cost-effectiveness, reduction or elimination of pain, rapid expulsion of inflammatory mucus, and minimal side effects. In essence, this invention accelerates the body's natural self-repair process, ensuring efficient and cost-effective replication and regeneration of tissues.

The human nervous system actively absorbs specially formulated solutions, it enters the body through mucous membranes and skin.

The simultaneous elevation of sodium ion concentration outside nervous cell membrane and the concentration of various amino acids in the human nervous system.

The regeneration process involves the swift breakdown and elimination of inflammatory pathogens, uric acid, free radicals, and other substances.

The immune system actually attacks the tumor cells on the one hand and helps the tumor grow on the other. X solution enters the cancer cells and inhibits the tumor by interfering with the DNA replication of rapidly dividing tumor cells, disrupting the cell membrane formation process of the cancer cells and exerting its cell killing activity.

Literature on destroying cell membranes of cancer cells such as: Dr. Duffy of the Harry Perkins Institute for Medical Research used bee and bumblebee venom from Australia to test the effects on clinical subtypes of breast cancer, showing that high concentrations of melittin can completely destroy the cell membrane of cancer cells within 60 minutes, and in the process of killing cancer cells, the concentration does not harm normal cells; X solution has the destructive effect of cancer cell membrane and is friendly to human cells.

In tumor suppression, a key point is to inject the formulation solution into the tumor center or to soak the tumor.

The melanoma case in the embodiment, removal of cancer cells without scarring; In addition, breast cancer by soaking the nipple for a long time, can quickly reduce breast fluid, hard mass shrink.

The method of inhibiting cancer with X solution can be widely used in the inhibition of gastric cancer, liver cancer, kidney cancer, pancreatic cancer and brain cancer.

Subsequently, guided by electrical signals from the nervous system, fat cells, stem cells, and regenerative nutrients adeptly and naturally converge near the site of injury, fostering the regeneration of human tissue. Throughout this process, it is imperative to consistently and regularly administer the formulated solution on a daily basis. The regenerative effects are characterized by scar-free healing, absence of pain and inflammation, and a regenerated outcome that strikingly resembles the original healthy tissue.

The simultaneous elevation of sodium ion concentration outside nervous cell membrane and the concentration of various amino acids in the human nervous system, reaching or surpassing levels found in the sodium ion concentration outside nervous cell membrane of octopuses, could potentially activate the regenerative capacity of the human body.

Skin and muscle regeneration is completed without scar repair and regeneration.

In the embodiment, the regeneration growth rate is slow, which is completed in two months. In addition, the leg tendons will show a reminder signal to remind the application of X solution, and there will be slight discomfort if it is not applied.

The inner mucosa, such as eyes, brain and so on, realizes regeneration, and the skin muscle realizes regeneration is very different; The repair process of internal organs mainly consists in the rapid decomposition of inflammatory substances; The nervous system and immune system help expel inflammatory mucus; Aggregation and aggregation of stem cells and fat into the regenerative repair area to complete the regeneration process.

Formula to inhibit keloid attention points: apply the formula solution more than twice a day; Use formula solution, do not apply alcohol, iodine, hydrogen peroxide and other disinfection water.

Disinfectants such as alcohol and hydrogen peroxide, which cause disturbances in electrical signals in the nervous system, may be part of the reason for scars.

The formula solution can be used directly in surgical procedures to inhibit viral and bacterial reproduction, inhibit pain.

In the embodiment, the oral solution was used continuously, and gradually, plaque was eliminated, dental nerve repair was completed, gingival muscle grew, and periodontitis was basically healed.

Formula solution to inhibit hypertension: It is recommended to atomize the lungs, drop the eyes, soak the mucous membrane, and use the whole body.

Formula solution to inhibit diabetes: It is recommended to atomize the lungs, drop the eyes, soak the mucous membrane, and use the whole body.

The system of claim 1, Wherein said formulation solution possesses strong lipophilic properties , enabling efficient decomposition of polycyclic aromatic hydrocarbons such as benzo[a]pyrene and disruption of the viscosity of heavy oil fouling.

Wherein said formulation solution is applied topically to various human organs, including the skin, eyes, mucous membranes, oral cavity, intestines, and hair. The human skin, mucous membranes, eyes, and lung membranes exhibit rapid absorption of the formulation solution, especially when actively absorbed by the nervous system. The nutritional components are efficiently taken up by diverse cell types. The skin and mucous membranes of the human body can absorb the formulation solution over a prolonged period and across a large surface area, ensuring a safe, non-invasive, and comfortable experience.

The active absorption of the formulation solution by the human nervous system mimics the extensive and sustained uptake of various small nutrient molecules by plant roots.

The human nervous system actively absorbs the formula solution, including the central nervous system, peripheral nervous system and peripheral nerves, which will actively absorb the formula solution to achieve nervous system drug delivery.

In embodiments, different people using X have similar fixed characteristics: time, signaling pathway, mucus discharge, pain regularity, etc. At the same time, the biological degradation rate of X is fast, mainly due to the active absorption of the nervous system, and it is very safe for healthy cells and tissues.

It's difficult for drugs to cross the blood-brain barrier into the brain; But drug delivery through the nervous system, from the eye to the brain, is very fast and effective.

Solution X is absorbed by the skin through a large number of peripheral receptors in the nervous system.

The system of claim 1, Wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration.

Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair. This process stimulates the regeneration of various tissues, including the skin, muscles, nerves, blood vessels, and bones. Additionally.

Wherein said formula elucidated the potential to expedite the recovery rate of elderly individuals_{∘}

Regenerative medicine is a widely recognized concept that endeavors to enhance the body's self-repair mechanisms to a clinically meaningful extent.

Currently, there are three internationally recognized regenerative medical technologies. The first one is cell therapy, The second type of regenerative medical technology utilizes foreign materials to stimulate the body's repair mechanisms. The third type of regenerative medical technology involves the utilization of devices that alleviate the body's burden and facilitate self-healing processes.

The simultaneous elevation of sodium ion concentration outside nervous cell membrane and the concentration of various amino acids in the human nervous system, reaching or surpassing levels found in the sodium ion concentration outside nervous cell membrane of octopuses, could potentially activate the regenerative capacity of the human body.

Distinctions between this application and the forefront of international regenerative medicine: This application sets itself apart in several ways. Firstly, it eliminates the need for ex vivo cultivation or injection of stem cells, making the process more convenient and accessible. Secondly, it avoids the requirement for small intestinal mucosa, reducing the level of artificial intervention. Instead, it focuses on targeted application to specific areas such as the skin, mucous membranes, pulmonary mucosa, intestinal mucosa, upper and lower eyelids, and retina. The formulated solution can be administered through nebulization, topical application, or immersion.

To tackle the challenges of aging and regeneration, the present invention offers a formula that inhibits aging and promotes repair. The formula is absorbed by the human nervous system and undergoes conversion into various neurotransmitters, generating high-level electrical signals that propagate along the established neural pathways. This allows for the route of administration through the fixed neural pathways. Simultaneously, the sodium ion concentration outside nervous cell membrane can increase from 150 mmol/l to 4524 mmol/l (the saturation concentration of sodium ions) before returning to 150 mmol/l. Through repeated daily use of the formula solution, a continuous and stable spiral curve is formed, ranging from 150 to 4524 and back to 150, enabling the regeneration and repair of human tissues and organs. During this spiral curve process, when the sodium ion concentration outside nervous cell membrane exceeds 440 mmol/l, which is observed in regenerative animals such as octopuses and squids, it triggers the regenerative capacity of humans. By continuously accumulating this regenerative capacity, it becomes possible to achieve the regeneration and repair of human tissues and organs.

Moreover, when the sodium ion concentration outside nervous cell membrane reaches 4524 mmol/l during the spiral curve process, surpassing the extracellular sodium ion concentration of malignant tumors at 451 mmol/l, the electrical signals generated at 4524 mmol/l can rapidly block pain signals caused by 451 mmol/l. This mechanism inhibits the generation of cancer-related pain. Additionally, it facilitates the rapid discharge of inflammatory mucus, suppresses the replication of viruses and bacteria, and inhibits inflammation and cancer. As a result, it effectively combats diseases associated with aging, achieving the desired effect of delaying the aging process.

This anti-aging and regenerative formula boasts minimal side effects, excellent efficacy, and a straightforward application process.

The process of repairing and regenerating brain and spinal cord nerves is considered difficult to achieve in traditional medicine. Early studies suggested that astrocytes produce neurotrophic factors (NTFs) to support the growth and development of neurons, while microglial cells can transform into phagocytes to remove degenerated neurons due to aging or disease. Astrocytes proliferate to fill the gaps left by dead neurons. However, aging and inflammation make the repair process difficult and inefficient, leading to the expansion of neural damage.

X solution can be applied to the eyes and actively absorbed by the nervous system, transmitting from the eyes to the brain. It continuously supplies small molecular substances to the brain, decomposing inflammatory substances and alleviating or eliminating inflammation. The inflammatory mucous travels through the exchange of cerebrospinal fluid, enters the meningeal lymphatic vessels, and is expelled from the posterior wall of the pharynx through peripheral lymphatic vessels. The amount of expelled mucous is correlated with the severity of inflammation: the more severe the inflammation, the more mucous is expelled, and vice versa. Supplementation of amino acids, lipids, and other small molecules promotes the increase of neurotransmitters and neurotrophic factors, which is beneficial for the repair of brain neurons.

The amino acid peptides in X solution are natural small molecular components of proteins that bind to corresponding receptors on nerve cells, promoting the increase of neurotransmitters and enhancing bioelectrical signaling.

In summary, X solution can deliver small molecular components to distant parts of the nervous system along the neural system. It simultaneously blocks or eliminates the transmission of inflammatory signals, leading to the regular secretion of mucus, regular pain suppression, regular bioelectrical signaling, and regular regenerative ability. These regularities greatly help the nervous system by promoting axonal regeneration, repairing damaged myelin sheaths, enhancing neuronal signal transmission, regenerating blood vessels, reducing inflammatory scars, and avoiding physical barriers to regeneration and repair.

Inhibiting brain neuronal aging and functional decline.

The brain consists of billions of neurons, trillions of synapses, and millions of kilometers of fibers. It consumes 60 watts of power, equivalent to the power consumption of a light bulb. The brain is highly intricate but fragile.

Firstly, neurons are most susceptible to hypoxia. When the axoplasmic transport responsible for the nutritional transport of neurons and the aerobic metabolism of nerve fibers are disrupted, ATP drops below 50%, and fast axoplasmic flow stops. ATP is the energy currency of the human body, with mitochondria producing 32 ATP in the presence of oxygen but only 2 ATP in hypoxic conditions. Hypoxia causes significant damage to neurons.

Secondly, aging weakens the functionality and stability of neurotrophic factors and lowers their penetration level. Known growth factors, such as NGF/BDNF/NT-3/NT-4/NT-5/NT-6/NT-7/GNTF, which can induce the survival, growth, and differentiation of neurons, have low stability and penetration levels.

Thirdly, aging leads to a decrease in neurotransmitter levels.

The essence of bioelectricity lies in the transmembrane flow of ions, mediated by neural synaptic channel proteins such as sodium pumps, sodium ion channels, calcium ion channels, potassium ion channels, acetylcholine receptors, glutamate receptors, and many other channel proteins (some of which have not been documented in scientific literature). This intricate network forms an electrical signaling system. However, due to factors like hypoxia, disease, and aging, the quantity of neurotransmitters decreases continuously.

The substances in the X solution, once actively absorbed by the nervous system, are transported to the synapses. The vesicles in the synapses engulf the components of the formula solution, supplementing them into the vesicles and synthesizing neurotransmitters. The ingredients of the formula solution are absorbed and converted into a diverse range of neurotransmitters, which are then utilized in signal transmission. Saturated concentrations of cations, anions, and various amino acids occupy the channels simultaneously, continuously delivering the components for an extended period. This impedes and suppresses pain signals, achieving the effect of pain inhibition, thereby facilitating both neuronal regeneration and pain suppression.

Alzheimer's disease, is a degenerative aging disease, the traditional treatment is to inhibit inflammatory amyloid beta protein deposition and TAU protein entanglement;

Continuous use of X solution, through the biological signal delivery from the eye to the brain, meningeal lymphatic vessels release inflammatory mucus and discharge a large amount of inflammatory mucus in the throat wall, which is an effective method to inhibit AD and other degenerative central nervous system diseases. X solution can accelerate the repair of optic nerve, continuous transmission of signals from eye to brain, promote the repair of axon damage of neurons, repair the information delivery pathway from eye to brain, and gradually repair brain tissue.

Human experiments have been carried out in the United States and Japan on the regeneration of articular cartilage. In Japan, stem cells were injected into the joint, and the effect was improved. Dull pain appeared a day later, and squatting was possible within a week.

For the regeneration of articular cartilage, X solution adopts the method of soaking mucous membranes and/or solution apply to the skin, non-invasive administration, and the effect is that pain disappears quickly and recovery is fast.

X advantage: Using soaked mucous membrane and skin application, non-invasive administration; Pain inhibition is faster; Supplement small peptide molecules to accelerate the breakdown of inflammatory substances; Regeneration, the nervous system will gather repair stem cells, continue to use the X solution for time accumulation, and gradually return to the appearance of health.

Example Skin and muscle regeneration experiment: transparent thin scab support function, is a way of regeneration repair; Thin scabs continue to slowly thicken, constantly raise the blood scabs, and make a fixed template for the muscle skin, slowly repair and regeneration without traces, a very intelligent regeneration and repair method.

Hyaluronic acid is a glycosaminoglycan, one of the components of the extracellular matrix. It has the functions of lubricating joints, regulating proteins, diffusion and operation of water electrolyte, etc. In particular, it has the effect of water retention. It has the ability to lock water and is an ideal moisturizing factor. After the cells absorb X formula solution, there are rich hydroxy-carboxyl group resources, which is conducive to the production of hyaluronic acid in the body.

The system of claim 1, Wherein said formulation solution effectively disrupts pain signal transmission and expeditiously eliminates excessive inflammatory mucus, while concurrently suppressing inflammation and tumors, resulting in pain relief. It also impedes the formation of scars and keloids, as well as the proliferation of viral and bacterial pathogens. The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair. Furthermore, it demonstrates inhibitory effects on conditions such as periodontitis, hypertension, diabetes, as well as the overall age-related decline in bodily functions, either individually or in various combinations.

The formula is nervous system friendly, travels along the nervous system, and can inhibit some hidden viruses.

Case of inhibiting herpes virus: the patient's body has herpes virus for 15 years, about two months of irregular hair, many small bubbles overgrow the back genitals, etc., the pain is unbearable; After soaking the genital mucosa and applying the formula solution, the pain decreased and disappeared, and the herpes atrophied and crusted; Does not appear after continuous daily use; But viruses like herpes can hide in the body and reappear when the body's immune system is down.

A prevailing belief among medical researchers is that cancer is fundamentally an immune system disorder, characterized by the failure of the immune system to identify and control the spread and migration of malignant tumors.

Malignant tumor cells exhibit an extracellular sodium ion concentration of 451 mmol/l, which closely resembles the extracellular sodium ion concentration found in regenerative neuronal cells of animals like octopuses and squids, which is around 440 mmol/l. Furthermore, malignant tumor cells demonstrate amino acid absorption rates that are more than ten times higher than those of normal cells. Simultaneously, the sodium ion concentration outside nervous cell membrane can increase from 150 mmol/l to 4524 mmol/l (the saturation concentration of sodium ions) before returning to 150 mmol/l. Through repeated daily use of the formula solution, a continuous and stable spiral curve is formed, ranging from 150 to 4524 and back to 150, enabling the regeneration and repair of human tissues and organs. During this spiral curve process, when the sodium ion concentration outside nervous cell membrane exceeds 440 mmol/l, which is observed in regenerative animals such as octopuses and squids, it triggers the regenerative capacity of humans. By continuously accumulating this regenerative capacity, it becomes possible to achieve the regeneration and repair of human tissues and organs.

Moreover, when the sodium ion concentration outside nervous cell membrane reaches 4524 mmol/l during the spiral curve process, surpassing the extracellular sodium ion concentration of malignant tumors at 451 mmol/l, the electrical signals generated at 4524 mmol/l can rapidly block pain signals caused by 451 mmol/l. This mechanism inhibits the generation of cancer-related pain. Additionally, it facilitates the rapid discharge of inflammatory mucus, suppresses the replication of viruses and bacteria, and inhibits inflammation and cancer. As a result, it effectively combats diseases associated with aging, achieving the desired effect of delaying the aging process.

Smaller melanomas can disappear in more than two months without scarring; Continuous use can inhibit cancer cells.

The advantages of X inhibiting cancer include: 1, little or no pain; Second, to protect the nervous system and prevent tumors from migrating along the nervous system; Third, the infiltration mechanism of cancer cells is destroyed, and the cancer cells cannot increase in value, while killing the bacteria inside the tumor.

Moreover, when the sodium ion concentration outside nervous cell membrane reaches 4524 mmol/l during the spiral curve process, surpassing the extracellular sodium ion concentration of malignant tumors at 451 mmol/l, the electrical signals generated at 4524 mmol/l can rapidly block pain signals caused by 451 mmol/l. This mechanism inhibits the generation of cancer-related pain. Additionally, it facilitates the rapid discharge of inflammatory mucus, suppresses the replication of viruses and bacteria, and inhibits inflammation and cancer.

Formula to inhibit keloid attention points: apply the formula solution more than twice a day; Use formula solution, do not apply alcohol, iodine, hydrogen peroxide and other disinfection water.

Disinfectants such as alcohol and hydrogen peroxide, which cause disturbances in electrical signals in the nervous system, may be part of the reason for scars.

The formula solution can be used directly in surgical procedures to inhibit viral and bacterial reproduction, inhibit pain.

In the embodiment, the oral solution was used continuously, and gradually, plaque was eliminated, dental nerve repair was completed, gingival muscle grew, and periodontitis was basically healed.

Formula solution to inhibit hypertension: It is recommended to atomize the lungs, drop the eyes, soak the mucous membrane, and use the whole body.

Formula solution to inhibit diabetes: It is recommended to atomize the lungs, drop the eyes, soak the mucous membrane, and use the whole body.

To inhibit periodontitis, hypertension, and diabetes, the main focus is to accelerate the breakdown of inflammation and eliminate the removal of inflammatory substances, while increasing the delivery of supplementary small molecule nutrients into the body to improve the ability of self-healing.

Due to aging, tissues and organs decline in all directions, and inflammation and tumor are the most important ways. Timely removal of inflammatory substances, inhibition of tumor and inhibition of inflammation are the key points of anti-aging. Supplementing small molecules of nutrients for repair and regeneration is essential.

The system of claim 1, Wherein said In practical applications, the formulation of the present invention is administered to any part of the human body for disease prevention or treatment. It can also be utilized for disease prevention or treatment in mammals and pets. The formulation of the present invention can be manufactured in various forms, including tablets, injections, nebulizers, ointments, patches, topical applications, acupoint patches, aerosol sprays, bottled liquids, capsules, and any other pharmaceutical production and manufacturing processes or their combinations.

The results of animal experiment and human experiment are similar in atomization and injection.

The splitting, addition, or substitution of formulations that can easily be thought of by any technical person familiar with the technical field within the technical scope of the present invention shall be covered by the protection of the present invention. Therefore, the scope of protection of the invention should be subject to the scope of protection of the claim.

## Claims

1. An anti-aging and regenerative formula the formula includes tea components and sodium chloride, potassium chloride, and any combination of sodium salts, potassium salts, and calcium salts permitted by any pharmaceutical; Tea has more than 500 ingredients, 500 tea ingredients, Vitamins (C, B1, B2, B3, B5, B6, B11, B12, P1, H, A, D, E, K Etc;), amino acids (theanine, aspartate, serine, glutamic acid, glycine, tryptophan, valine, histidine, arginine, threonine, alanine, proline, cysteine, tyrosine, methionine, lysine, isoleucine, leucine, phenylalanine, etc;), alkaloids (caffeine, tealeine, theobromine, etc;), tea polyphenols (catechin, flavonoids, flowers, etc;) Pigments, phenolic acids, etc;), inorganic substances (potassium, phosphorus, calcium, magnesium, iron, sulfur, aluminum, fluorine, etc;), organic acids, lipids (phospholipids, triglycerides, sulfur lipids, glycolipids), pigments (chlorophyll, anthocyanin, carotene, lutein, etc;), aromatic substances, enzymes; One of the formulation of the solution includes Tieguanyin tea, sodium chloride, and water; the tea is boiled to produce a high concentration of tea, while simultaneously achieving a state of saturation in the presence of sodium chloride (reaching the maximum saturation level of sodium chloride dissolution);
Wherein said formulation solution possesses strong lipophilic properties , enabling efficient decomposition of polycyclic aromatic hydrocarbons such as benzo[a]pyrene and disruption of the viscosity of heavy oil fouling; Wherein said formulation solution is applied topically to various human organs, including the skin, eyes, mucous membranes, oral cavity, intestines, and hair; The human skin, mucous membranes, eyes, and lung membranes exhibit rapid absorption of the formulation solution, especially when actively absorbed by the nervous system; The nutritional components are efficiently taken up by diverse cell types; The skin and mucous membranes of the human body can absorb the formulation solution over a prolonged period and across a large surface area, ensuring a safe, non-invasive, and comfortable experience;
The active absorption of the formulation solution by the human nervous system mimics the extensive and sustained uptake of various small nutrient molecules by plant roots;
Wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration;
Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair; This process stimulates the regeneration of various tissues, including the skin, muscles, nerves, blood vessels, and bones; Additionally;
Wherein said formulation solution effectively disrupts pain signal transmission and expeditiously eliminates excessive inflammatory mucus, while concurrently suppressing inflammation and tumors, resulting in pain relief; It also impedes the formation of scars and keloids, as well as the proliferation of viral and bacterial pathogens; The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair; Furthermore, it demonstrates inhibitory effects on conditions such as periodontitis, hypertension, diabetes, as well as the overall age-related decline in bodily functions, either individually or in various combinations.

2. The system of claim 1, Wherein said formulation solution possesses strong lipophilic properties , enabling efficient decomposition of polycyclic aromatic hydrocarbons such as benzo[a]pyrene and disruption of the viscosity of heavy oil fouling;
Wherein said formulation solution is applied topically to various human organs, including the skin, eyes, mucous membranes, oral cavity, intestines, and hair; The human skin, mucous membranes, eyes, and lung membranes exhibit rapid absorption of the formulation solution, especially when actively absorbed by the nervous system; The nutritional components are efficiently taken up by diverse cell types; The skin and mucous membranes of the human body can absorb the formulation solution over a prolonged period and across a large surface area, ensuring a safe, non-invasive, and comfortable experience;
The active absorption of the formulation solution by the human nervous system mimics the extensive and sustained uptake of various small nutrient molecules by plant roots.

3. The system of claim 1, wherein said formulation solution promotes the body's regenerative processes and enhances the rate of anti-aging by fulfilling the following reparative conditions: suppressing the decline of various amino acids, vitamins, and other small molecules; inhibiting the reduction of neurotransmitters; preventing the depletion of neurotrophic factors and vital nutrients for neural glial cells; and simultaneously increasing the sodium ion concentration outside nervous cell membrane and various neurotransmitters to facilitate repair and regeneration;
Wherein said formulation solution is applied to activate the regenerative function in the human body, enabling the accumulation and mobilization of adipose tissue, reparative nutrients, and neural stem cells through the immune system, lymphatic tissues, and nervous system, targeting the specific sites that require regeneration and repair; This process stimulates the regeneration of various tissues, including the skin, muscles, nerves, blood vessels, and bones.

4. The system of claim 1, wherein said formulation solution effectively disrupts pain signal transmission and expeditiously eliminates excessive inflammatory mucus, while concurrently suppressing inflammation and tumors, resulting in pain relief; It also impedes the formation of scars and keloids, as well as the proliferation of viral and bacterial pathogens; The mentioned anti-aging and regenerative properties encompass the ability to address at least one disease from the following categories of inflammatory and tumorous conditions found in human tissues and organs: eyes, brain, nervous system, lungs, skeletal system, blood vessels, muscles, skin, teeth and gums, lymphatic system, pancreas, liver, kidneys, and hair; Furthermore, it demonstrates inhibitory effects on conditions such as periodontitis, hypertension, diabetes, as well as the overall age-related decline in bodily functions, either individually or in various combinations.

5. The system of claim 1, Wherein said In practical applications, the formulation of the present invention is administered to any part of the human body for disease prevention or treatment; It can also be utilized for disease prevention or treatment in mammals and pets; The formulation of the present invention can be manufactured in various forms, including tablets, injections, nebulizers, ointments, patches, topical applications, acupoint patches, aerosol sprays, bottled liquids, capsules, and any other pharmaceutical production and manufacturing processes or their combinations.
